# EUROPEAN PATENT SPECIFICATION

(11) **EP 2 037 902 B1**
(45) Date of publication and mention of the grant of the patent: **03.07.2013**
(21) Application number: 07733311.0
(22) Date of filing: 21.06.2007
(51) Int. Cl.: A61K 31/05, A61K 31/352, A61P 25/02, A61P 29/00

(54) **CANNABINOIDS FOR USE IN THE TREATMENT OF NEUROPATHIC PAIN**
CANNABINOIDE ZUR VERWENDUNG BEI DER BEHANDLUNG VON NEUROPATHISCHEN SCHMERZEN
CANNABINOÏDES DESTINÉS À ÊTRE UTILISÉS DANS LE TRAITEMENT DE LA DOULEUR NÉVROPATHIQUE

(30) Priority: 23.06.2006 GB 0612512
(43) Date of publication of application: 25.03.2009
(73) Proprietor: GW Pharma Limited, Salisbury Wiltshire SP4 0JR (GB)
(72) Inventor: GUY, Geoffrey, Salisbury Wiltshire SP4 0JR (GB); COSTA, Barbara, 20126 Milan (IT)
(74) Representative: Schiller, Dominic Christopher
(86) International application number: PCT/GB2007/002315
(87) International publication number: WO 2007/148094

(56) References cited:
- WO-A-03/037306
- GB-A- 2 394 894
- BARNES MICHAEL PHILIP: "Sativex: clinical efficacy and tolerability in the treatment of symptoms of multiple sclerosis and neuropathic pain" EXPERT OPINION ON PHARMACOTHERAPY, ASHLEY, LONDON,, GB, vol. 7, no. 5, April 2006 (2006-04), pages 607-615, XP009088410 ISSN: 1465-6566
- PERRAS C: "Sativex for the management of multiple sclerosis symptoms" ISSUES IN EMERGING HEALTH TECHNOLOGIES, CANADIAN COORDINATING OFFICE FOR HEALTH TECHNOLOGY ASSESSMENT,, CA, no. 72, 2 September 2005 (2005-09-02), pages 1-4, XP009088416 ISSN: 1488-6316
- RUSSO ET AL: "A tale of two cannabinoids: The therapeutic rationale for combining tetrahydrocannabinol and cannabidiol" MEDICAL HYPOTHESES, EDEN PRESS, PENRITH, US, vol. 66, no. 2, February 2006 (2006-02), pages 234-246, XP005198767 ISSN: 0306-9877
- NURMIKKO TURO J ET AL: "A MULTI-CENTRE, DOUBLE-BLIND, RANDOMIZED, PLACEBO-CONTROLLED TRIAL OF ORO-MUCOSAL CANNABIS-BASED MEDICINE IN THE TREATMENT OF NEUROPATHIC PAIN CHARACTERIZED BY ALLODYNIA" NEUROLOGY, LIPPINCOTT WILLIAMS & WILKINS, PHILADELPHIA, US, vol. 64, no. 6 SUPPL 1, 19 April 2005 (2005-04-19), page A374, XP009076274 ISSN: 0028-3878
- PEREZ JORDI: "COMBINED CANNABINOID THERAPY VIA AN OROMUCOSAL SPRAY" DRUGS OF TODAY / MEDICAMENTOS DE ACTUALIDAD, J.R. PROUS SS.A. INTERNATIONAL PUBLISHERS, ES, vol. 42, no. 8, August 2006 (2006-08), pages 495-503, XP009076360 ISSN: 0025-7656

## Description

The present invention relates to the use of cannabidiol (CBD) and tetrahydrocannabinol (THC) for use in the treatment of neuropathic pain. The ratio of CBD to THC is between 18:1 and 30:1. More preferably the CBD and THC are in the form of plant extracts.

### BACKGROUND TO THE INVENTION

Pain is one of the most common reasons for a patient to seek medical care and in consequence, pain results in a tremendous number of lost work days per year.

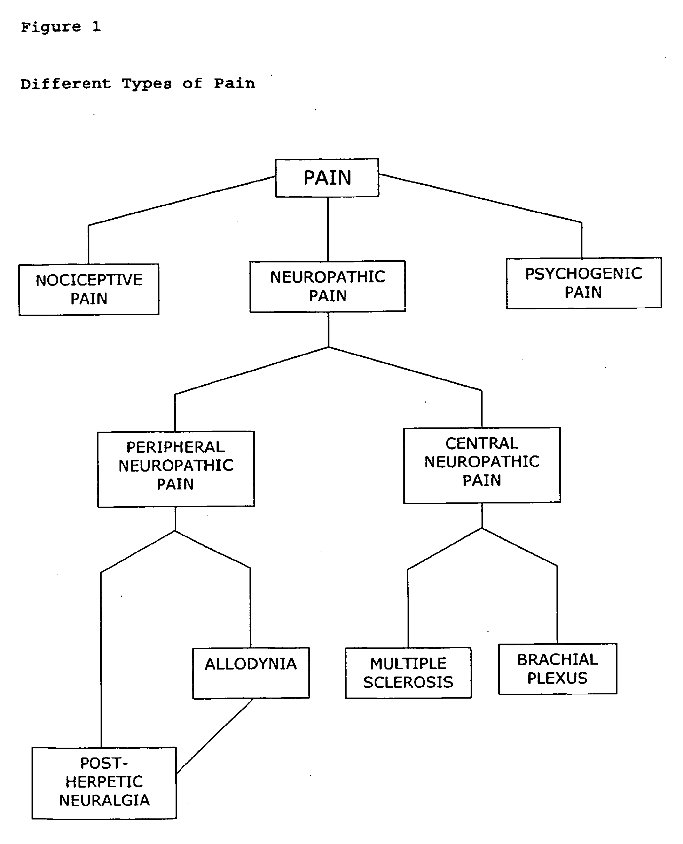

There are three general classes of pain: nociceptive pain, neuropathic pain, and psychogenic pain. Figure 1 describes the different types of pain and how certain types of diseases such as allodynia and multiple sclerosis are classified.

In nociceptive pain, the stimulation of the sensory nerve endings called nociceptors causes the sensation of pain. Such pain often occurs after injury or surgery. The pain signals are transmitted by the nociceptors to the brain. Often the pain is localised, constant and has an aching or throbbing quality. Once the damage to the tissue heals the pain usually resolves. Treatment with opioids may resolve nociceptive pain.

Psychogenic pain, is a pain disorder that is associated with psychological factors. Some types of mental or emotional problems can cause pain. They can also increase or prolong pain. Headaches, muscle pains, back pain, and stomach pains are some of the most common types of psychogenic pain. People with this pain disorder actually have real pain. The diagnosis is made when all physical causes of pain are ruled out.

Neuropathic pain is the result of an injury or malfunction of the peripheral or the central nervous system. The pain may be triggered by an injury but not necessarily by an injury of the nervous system itself. Neuropathic pain is frequently chronic and is often less responsive to treatment with opioids, but may respond to treatment with anticonvulsant or antidepressant drugs.

Neuropathic pain is caused by abnormalities in the nerves, spinal cord or brain and is a chronic type of non-malignant pain with an estimated prevalence of over 1% of the population. Optimising pain relief in these patients is crucial in helping a patient regain control of his or her life.

The most common cause of neuropathic pain is injury or dysfunction of nerves. Injury or dysfunction of peripheral nerves or nerves descending from the spinal cord results in disinhibition of nerve impulses at the spinal cord which in consequence results in pain. Neuropathic pain can also be centrally mediated, rather than peripheral, in conditions such as spinal cord injury and multiple sclerosis.

Neuropathic pain can therefore be divided into two further classes; peripheral neuropathic pain and central neuropathic pain depending on whether the peripheral or central nervous system is affected.

Patients with peripheral neuropathic pain often experience pain which feels like a burning or electrical pain, whereas others describe their pain as feeling like extreme cold or pins and needles.

The pain may be worsened by activity or by wearing clothes over the affected area. The pain may also follow a daily pattern, which may mean it is worse at certain times of the day.

Allodynia is a type of peripheral neuropathic pain. This is a painful response to a typically non-painful stimulus, for example brushing the affected area with a fingertip. The pain tends to increase with repeated stimulation and may spread from the affected area. Allodynic pain can be evoked in response to chemical, thermal (cold or heat) or mechanical low or high intensity stimuli applied either statically or dynamically to skin, joints, bone, muscle or viscera. It is thought that the presence of allodynic pain is a more suitable means of grouping patients suffering from peripheral neuropathic pain than by the specific disease that led to the neuropathic pain.

It is clear that patients that suffer from neuropathic pain can have their quality of life greatly affected by it. The pain can interfere with work and social activities as well as with the amount and quality of sleep that a patient experiences. A successful treatment for the relief of neuropathic pain should improve both the amount of pain that the patient is experiencing as well as improving the patient's quality of life.

Non-pharmaceutical methods of treating neuropathic pain include transcutaneous electrical nerve stimulation (TENS) and acupuncture.

The use of pharmaceuticals is the most common treatment for neuropathic pain. These include topical creams applied directly to the site of pain. Analgesics, antidepressants and anticonvulsants are the other drug classes generally in use. The drug carbamezepine, which is an anticonvulsant, is currently the only FDA approved drug which has an indication for neuropathic pain. It has been suggested in post-marketing studies that there is a five- to eight-fold increase in the risk of blood dyscrasias in patients taking carbamezepine. In 7% of patients there has been shown to be a 25% decrease in their white blood cell count, this usually reverses within the first 4 months of therapy.

The use of cannabis as a medicine has long been known and during the 19^{th} Century, preparations of cannabis were recommended as a hypnotic sedative which were useful for the treatment of hysteria, delirium, epilepsy, nervous insomnia, migraine, pain and dysmenorrhoea.

Until recent times the administration of cannabis to a patient could only be achieved by preparation of cannabis by decoction which could then be swallowed, or by the patient inhaling the vapours of cannabis by smoking the dried plant material. Recent methods have sought to find new ways to deliver cannabinoids to a patient including those which bypass the stomach and the associated first pass effect of the liver which can remove up to 90% of the active ingested dose and avoid the patient having to inhale unhealthy tars and associated carcinogens into their lungs.

Formulations containing specific, defined ratios of cannabinoids may be formulated from pure, synthetic cannabinoids or from extracts derived from the cannabis plant in combination with pharmaceutical carriers and excipients.

Cannabinoids are a group of chemicals known to activate cannabinoid receptors in cells. These chemicals, which are found in cannabis plants, are also produced endogenously in humans and other animals, these are termed endocannabinoids. Synthetic cannabinoids are chemicals with similar structures to plant cannabinoids or endocannabinoids.

Some plant cannabinoids can also be purified to such an extent that all of the other naturally occurring compounds, such as, other minor cannabinoids and molecules such as terpenes are removed. This purification results in a purity of greater than 99% (w/w) of the target cannabinoid. To a certain extent, these purified cannabinoids can be considered to be the same as synthetic cannabinoids as they consist only of the target cannabinoid.

It has been shown previously that the cannabinoid cannabidiol (CBD) administered as a purified compound can partially relieve neuropathic pain (Costa et al., 2004). This was shown using the neuropathic pain model of chronic constriction injury of the rat sciatic nerve and testing the effectiveness of the test article with thermal and mechanical hyperalgesia and mechanical allodynia. These animal models are used to predict the effectiveness of a test compound on neuropathic pain.

Neuropathic pain is often associated with a diverse and complex set of pain stimuli and as such is difficult to treat effectively as the response to treatment is unpredictable.

Surprisingly, the applicants have found that administration of the cannabinoids cannabidiol (CBD) and delta-9-tetrahydrocannabinol (THC) is more efficacious in the treatment of neuropathic pain than either of the cannabinoids CBD or THC alone.

In particular the cannabinoids CBD and THC were in a ratio of approximately 24:1 (CBD:THC).

Sativex R is a commercially available CBME comprising cannabidiol and THC in a ratio of 1:1 known for the treatment of neuropathic pain see e.g. BARNES MICHAEL PHILIP: "Sativex: clinical efficacy and tolerability in the treatment of symptoms of multiple sclerosis and neuropathic pain" EXPERT OPINION ON PHARMACOTHERAPY, ASHLEY, LONDON,, GB, vol. 7, no. 5, April 2006 (2006-04), pages 607-615, XP009088410 ISSN: 1465-6566 and NURMIKKO TURO J ET AL: "A MULTI-CENTRE, DOUBLE-BLIND, RANDOMIZED, PLACEBO-CONTROLLED TRIAL OF ORO-MUCOSAL CANNABIS-BASED MEDICINE IN THE TREATMENT OF NEUROPATHIC PAIN CHARACTERIZED BY ALLODYNIA" NEUROLOGY, LIPPINCOTT WILLIAMS & WILKINS, PHILADELPHIA, US, vol. 64, no. 6 SUPPL. 1, 19 April 2005 (2005-04-19), page A374, XP009076274 ISSN: 0028-3878.

### SUMMARY OF INVENTION

According to the first aspect of the present invention there is provided a cannabis based medicinal extract (CBME) comprising CBD and THC for use in the treatment of peripheral neuropathic pain, wherein the ratio of CBD to THC by weight is between 18:1 and 30:1.

The type of neuropathic pain is peripheral neuropathic pain.

Many different types of peripheral neuropathic pain are known to exist. Examples of diseases and syndromes that result in peripheral neuropathic pain include but are not limited to the following:
Hereditary disorders such as Charcot-Marie-Tooth disease and Friedreich's ataxia.
Systemic or metabolic disorders such as allodynia, peripheral herpetic neuralgia, diabetic neuropathy, dietary deficiencies (in particular deficiency in Vitamin B12), alcoholic neuropathy, uremia, cancer.
Infectious or inflammatory conditions such as AIDS, hepatitis, Colorado tick fever, diptheria, Guillian-barre syndrome, HIV infection, leprosy, Lymes disease, polyarteritis nodosa, rheumatoid artritis, sarcoidosis, Sjogren syndrom, syphilis, systemic lupus erythematosus and amyloid.
Exposure to toxic compounds such as sniffing glue or other toxic compounds, nitrous oxide, industrial agents - in particular solvents, heavy metals (lead, arsenic, mercury etc.) and neuropathy secondary to drugs.
Miscellaneous causes such as ischemia (decreased oxygen/decreased blood flow) and prolonged exposure to cold temperature

Preferably the THC and CBD are derived from plant extracts.

The compositions of plant extracts are described in Table 1. Cannabinoid-containing plant extracts will often comprise the major cannabinoid and a minor cannabinoid, along with several other cannabinoids. There will also be a non-cannabinoid fraction that will often contain components such as terpenes and other minor plant derived components including: sterols, triglycerides, alkanes, squalene, tocopherol and carotenoids.

For example a THC-containing plant extract may comprise between 63 and 78 %(w/w) THC in addition to CBD at 0.1-2.5%(w/w). The other cannabinoids include: cannabigerol (1.0-2.0%(w/w)), cannabichromene (0.8-2.2%(w/w)), tetrahydrocannabidivarin (0.4-1.0%(w/w)), and tetrahydrocannabinolic acid (<2.0%(w/w)). The non-cannabinoid fraction may comprise monoterpenes (0.7%(w/w)), di/tri-terpenes (0.6%(w/w)), sesquiterpenes 1.7%(w/w)), other terpenes (<3.0%(w/w)), and other minor plant components at between 6.3 and 26.7 %(w/w).

Furthermore a CBD-containing plant extract may comprise between 57 and 72 %(w/w) CBD in addition to THC at 2.0-6.5%(w/w). The other cannabinoids include: cannabigerol (0.8-6.5%(w/w)), cannabichromene (3.0-6.5%(w/w)), cannabidivarin (1.0-2.0%(w/w)), and cannabidiolic acid (<2.0%(w/w)). The non-cannabinoid fraction may comprise monoterpenes (0.4%(w/w)), di/tri-terpenes (0.4%(w/w)), sesquiterpenes 2.0%(w/w)), other terpenes (<3.0%(w/w)), and other minor plant components at between 1.7 and 28.4%(w/w).

Preferably, the ratio of CBD and THC is between 20:1 and 28:1 (CBD:THC) by weight. More preferably is between 22:1 and 26:1 (CBD:THC) by weight. More preferably still, the ratio is about 24:1 (CBD:THC) by weight.

Favourably the CBD and THC are packaged for delivery in a titratable dosage form.

The CBD may be administered separately, simultaneously or sequentially to the THC.

For example the administration of CBD and THC to a patient could occur at the same time, wherein the CBD and THC would be contained in the same formulation. The cannabinoids could also be administered at separate times for example; a formulation containing CBD could be administered to a patient at a fixed time prior to a formulation containing THC in order to ameliorate some of the side effects of THC, which CBD is known to improve or vice versa. The two cannabinoids could also be administered consecutively to a patient if required.

The term "titrate" is defined as meaning that the patient is provided with a medication that is in such a form that smaller doses than the unit dose can be taken.

A "unit dose" is herein defined as a maximum dose of medication that can be taken at any one time or within a specified dosage period such as for example, 3 hours.

The titration of a unit dose is beneficial to the patient as they are often able to take smaller doses of the medication to obtain efficaciousness. It is understandable that not all patients will require exactly the same dose of medication, for example patients of a larger build or faster metabolism may require a higher dose than that required by a patient that is of a smaller build. Different patients may also present with different degrees of complaints and as such may require larger or smaller doses in order to treat the complaint effectively. The benefits of a titratable dosage form over dosage forms where smaller, incremental doses are difficult to take are therefore evident.

Unit dose ranges are preferably in the range of between 10 and 150 mg of the cannabinoid CBD, more preferably in the range of 50 to 100 mg, more preferably still in the range of 75 to 85 mg of CBD.

Unit dose ranges are preferably in the range of between 1 and 10mg of the cannabinoid THC, more preferably in the range of 2.5 to 5 mg, more preferably still the unit dose is approximately 4 mg of THC.

Preferably the maximum daily dosage dose of medicament is less than or equal to 1000 mg CBD.

Preferably the maximum daily dosage dose of medicament is less than or equal to 45 mg THC.

Preferably the pharmaceutical formulations are packaged for delivery such that delivery is targeted to an area selected from one or more of the following: sublingual; buccal; parenteral; oral; rectal, nasal; and the pulmonary system.

More preferably the pharmaceutical formulations are in the form selected from one or more of the following: gel; gel spray; tablet; liquid; capsule and for vaporisation. Additionally the pharmaceutical formulation further comprises one or more carrier solvents. Preferably the carrier solvents are ethanol and/or propylene glycol. More preferably the ratio of ethanol to propylene glycol is between 4:1 and 1:4. More preferably still the ratio is substantially 1:1.

Preferably the CBD and THC are present as a cannabis based medicine extract (CBME).

In order to accurately control the ratio of CBD and THC in the medicament a ratioed product is formulated from:
- a cannabis based medicinal extract which comprises THC at more than 90% of the total cannabinoid content in the extract; and
- a cannabis based medicinal extract which comprises CBD at more than 90% of the total cannabinoid content in the extract.

These are then mixed to the specified ratios.

In one embodiment the CBME are produced by extraction with supercritical or subcritical CO₂. In an alternative embodiment the CBME are produced by extraction from plant material by volatilisation with a heated gas. Preferably the CBME contains all of the naturally occurring cannabinoids in the plant material.

According to a second aspect of the present invention there is provided a CBME comprising CBD a THC for use in the treatment of neuropathic pain, wherein the ratio of CBD to THC by weight is between 18:1 and 30:1, for use in combination with one or more other medicinal substances.

Preferably the one or more other medicinal substances are one or more analgesic drugs.

More preferably, still the one or more other medicinal substances are one or more opiate or opiate related drugs.

Opiate or opiate related drugs include but are not limited to drugs chemically related to morphine and also non-related structures that act at the opioid receptors in the brain.

Preferably the one or more other medicinal substances are one or more anticonvulsant drugs.

Preferably the one or more other medicinal substances are one or more antidepressant drugs.

More preferably the CBD and THC are administered separately, simultaneously or sequentially to the one or more other medicinal substances.

The different therapeutic classes of medications that are useful to be used in addition to the combination of cannabinoid-containing plant extracts include but are not limited to: natural opium alkaloids, anti-epileptics, non-selective monoamine reuptake inhibitors, opioids, anilides, diphenylpropylamine derivatives, acetic acid derivatives and related substances, platelet aggregation inhibitors excluding heparin, carboxamide derivatives, propionic acid derivatives, salicylic acid derivatives, local anaesthetics, non-steroidal anti-inflammatory or anti-rheumatic compounds, coxibs, topical non-steroidal anti-inflammatory compounds, opium alkaloids and derivatives, anaesthetics for topical use, drugs used in opioid dependence, hydantoin derivatives, oripavine derivatives, phenylpiperidine derivatives.

According to a third aspect of the present invention there is provided a CBME comprising CBD and THC for use in the treatment of peripheral neuropathic pain, wherein the ratio of CBD to THC by weight is between 18:1 and 30:1, which additionally involves the treatment of sleep disturbance caused by peripheral neuropathic pain.
Figure 1 shows a diagram describing of the different types of pain;
Figure 2 shows a graph of the effect of acute administration of vehicle or test article in thermal hyperalgesia;
Figure 3 shows a graph of the effect of acute administration of vehicle or test article in mechanical allodynia;
Figure 4 shows a graph of the effect of repeated administration of vehicle or test article in thermal hyperalgesia; and
Figure 5 shows a graph of the effect of repeated administration of vehicle or test article in mechanical allodynia.

### SPECIFIC DESCRIPTION

There is a significant requirement for drugs that are able to efficiently treat neuropathic pain, which is a debilitating chronic pain that is refractory to many drugs.

In Example 1 described below, two animal models of neuropathic pain were used. Such methodologies are acknowledged to be a good test of neuropathic pain. In such models an assessment of the animals after nerve injury ensures the models are related to neuropathic pain.

Behaviours such as hyperalgesia, where there is a strong withdrawal response to a moderate heat stimulus and allodynia, where there is a strong withdrawal response to non-noxious tactile stimuli, are tested after a nerve injury. At the site of a nerve injury, the nerve fibres develop abnormal excitability. Persistent excitability then often spreads to distant parts of the peripheral and central nervous system.

The example describes an experiment comparing the efficacy of cannabinoid-containing plant extracts in comparison to purified cannabinoids in the treatment of neuropathic pain.

The compositions of a THC-containing plant extract and a CBD-containing plant extract are described in Table 1 below.

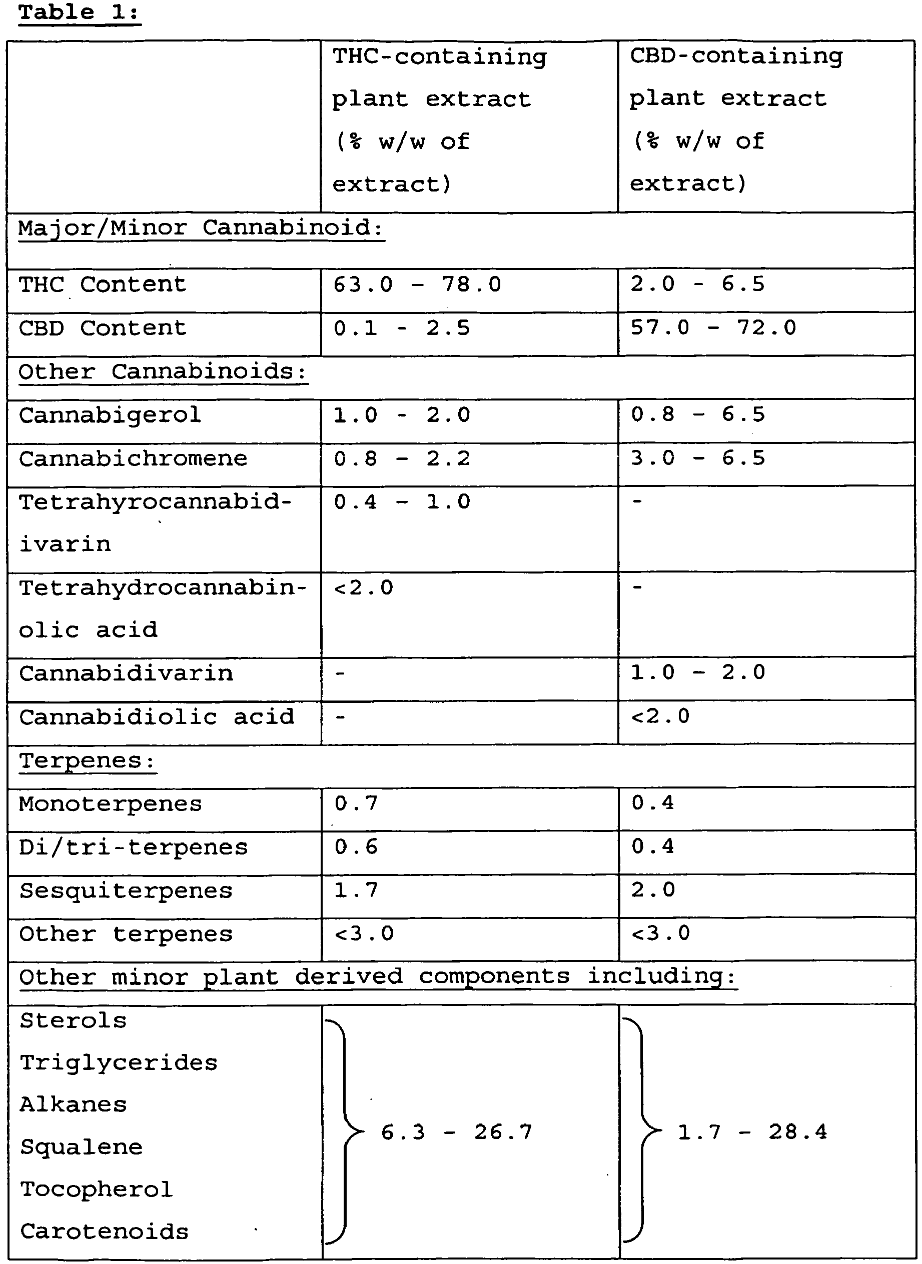

The features of the invention are illustrated further by reference to the following examples:

### Example 1:

### Effect of a Cannabis sativa extract on nociceptive behaviour in a rat model of neuropathic pain

Painful neuropathy was induced in male Wistar rats weighing 200-220 g (Harlan, Italy). Animals were anaesthetized with sodium pentobarbital (60 mg kg⁻¹ i.p.) and submitted to a surgical procedure to induce neuropathic pain according to Bennet & Xie (1988).

This procedure was a chronic constriction injury (CCI) to the sciatic nerve, this was achieved by exposing the common sciatic nerve at the level of the mid thigh and, proximal to the sciatic nerves trifurcation, four ligatures were loosely tied around it with about 1 mm spacing so that the epineural circulation was preserved.

Sham animals (sciatic exposure without ligation) were used as controls.

The compounds tested were a cannabinoid-containing plant extract which contained a ratio of CBD:THC of approximately 24:1. From Table 1 this could also be described as a "CBD-containing plant extract". The extract comprised CBD at a concentration of 10 mg/kg and THC at a concentration of 0.42 mg/kg. Pure cannabinoids CBD (at a concentration of 10 mg/kg) and THC (at a concentration of 0.42 mg/kg) were also used as test articles for comparison to the cannabinoid-containing plant extract.

The test compounds were dissolved in a 1:1:18 mixture of ethanol, cremophor and saline.

The acute administration of compounds was evaluated in rats treated with vehicle for one week and subsequently provided with the test article prior to the behavioural evaluations. The behavioural evaluations were performed at different times (between 30 min to 24 hours).

In the repeated administration study the rats received the test compounds or the vehicle orally once a day for seven days, starting from the seventh day after the surgical procedure.

The animals pain response was monitored (i) before surgery, (ii) on day 7 (before starting the treatment) and (iii) on day 14 (24 hours after the last administration of the test article).

Thermal hyperalgesia was tested according to the Hargreaves procedure (Hargreaves et al., 1988) using the plantar test (Ugo Basile, Varese, Italy).

Briefly, animals were placed in a clear plexiglass box and allowed to acclimatise. A constant intensity radiant heat source was aimed at the midplantar area of the hind paw. The time, in seconds, from initial heat source activation until paw withdrawal was recorded.

Mechanical allodynia was assessed using the Dynamic Plantar Aesthesiometer (Ugo Basile, Varese, Italy). Particularly, animals were placed in a test cage with a wire mesh floor, and the tip of von Frey-type filament was applied to the middle of the plantar surface of the hind paw. The filament exerted an increasing force starting below the threshold of detection and increasing until the animal removed its paw. Withdrawal threshold was expressed as tolerance level in g.

### Results:

### 1. Effect of acute administration of test article on thermal hyperalgesia

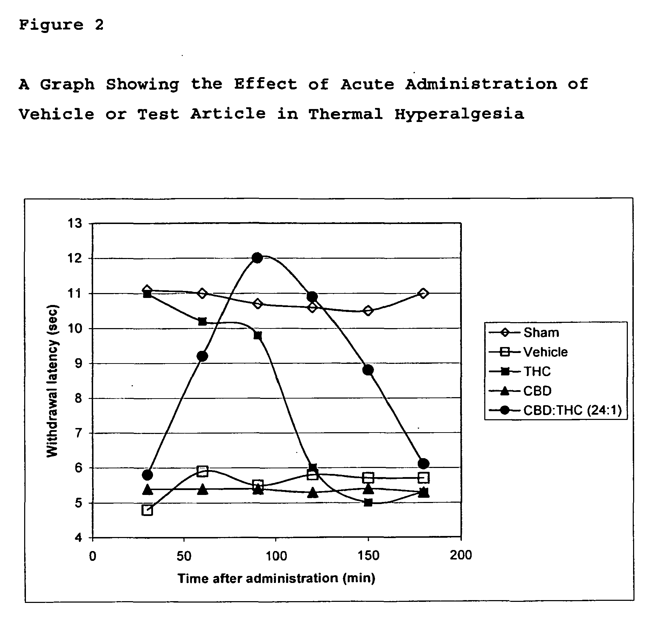

Table 2 below details the data produced by the acute administration of the vehicle to the sham animals or administration of vehicle or test article to animals with CCI, 14 days after the injury occurred. Figure 2 shows the effect of the acute administration of the test article or vehicle on thermal hyperalgesia.

**Table 2:**

| **Test Article** | **Time after administration (min)** | | | | | |
|---|---|---|---|---|---|---|
| | 30 | 60 | 90 | 120 | 150 | 180 |
| | **Withdrawal latency (sec)** | | | | | |
| Sham | 11.1 | 11.0 | 10.7 | 10.6 | 10.5 | 11.0 |
| Vehicle | 4.8 | 5.9 | 5.5 | 5.8 | 5.7 | 5.7 |
| THC | 11.0 | 10.2 | 9.8 | 6.0 | 5.0 | 5.3 |
| CBD | 5.4 | 5.4 | 5.4 | 5.3 | 5.4 | 5.3 |
| CBD:THC (24:1) | 5.8 | 9.2 | 12.0 | 10.9 | 8.8 | 6.1 |

The withdrawal latency gives an indication of the amount of pain that an animal is in. For example when an animal withdraws its paw soon after the heat source is applied it infers that the animal is in more pain than one that withdraws its paw a longer time after the heat source was applied.

As can be seen from Table 2 and Figure 2 the sham animals, (where no nerve injury had taken place), maintained a high withdrawal latency of around 11 seconds over the entire testing period. When these data are compared to the other test animals, which all had CCI, it can be seen that the CCI caused the animals to feel more pain.

In the CCI animals treated with vehicle the withdrawal latency was around 5.5 seconds over the entire testing period.

The three test articles pure THC, pure CBD and cannabinoid-containing plant extract containing CBD:THC (24:1) were shown to produce very different results.

As can be seen in Table 2 and Figure 2, the pure THC increased the withdrawal latency over that of the vehicle treated animals. The pain reliving effect of the pure THC began as early as 30 minutes after administration at the point of the first behavioural evaluation. The withdrawal latency was heightened over that of the vehicle treated animal but the withdrawal latency did not reach the same level as the sham animals. The pain relieving effect decreased over the study period and 120 minutes after administration there was no difference in the withdrawal latency of the vehicle and the pure THC.

The pure CBD did not produce any pain relieving effects over the study period as the withdrawal latency was very similar to that of the vehicle treated animals.

The withdrawal latency of the animals treated with the cannabinoid-containing plant extract containing CBD:THC (24:1) showed a steady increase of withdrawal latency over the first 90 minutes of the test period resulting in a withdrawal latency in excess of that produced by both the sham animals and the animals treated with pure THC. This shows that the cannabinoid-containing plant extract was more efficacious at relief of pain than the pure THC and was able to produce a longer profile of pain relief than any of the other test articles.

After 150 minutes of behavioural evaluation the pain relieving effect of the cannabinoid-containing plant extract containing CBD:THC (24:1) decreased and after 180 minutes the withdrawal latency for these animals was similar to that of all the other CCI animals.

### 2. Effect of acute administration of test article on mechanical allodynia

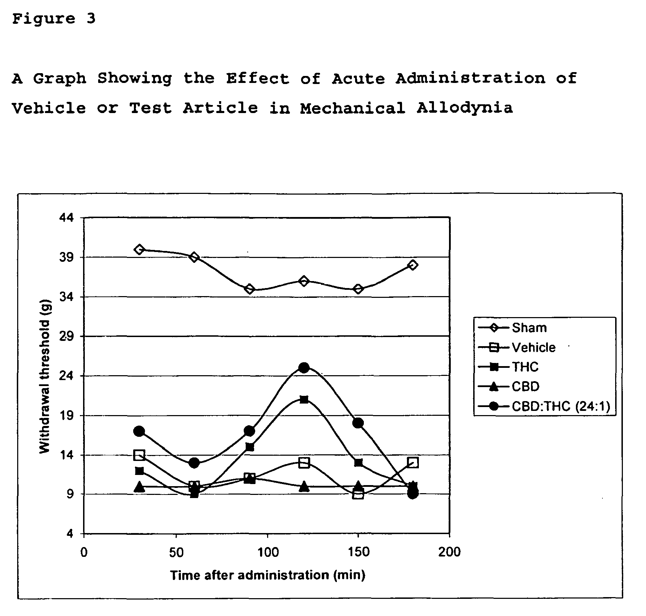

Table 3 below details the data produced by the acute administration of the vehicle to the sham animals or administration of vehicle or test article to animals with CCI, 14 days after the injury occurred. Figure 3 describes these data in a graphical form.

**Table 3:**

| **Test Article** | **Time after administration (min)** | | | | | |
|---|---|---|---|---|---|---|
| | 30 | 60 | 90 | 120 | 150 | 180 |
| | **Withdrawal threshold (g)** | | | | | |
| Sham | 40 | 39 | 35 | 36 | 35 | 38 |
| Vehicle | 14 | 10 | 11 | 13 | 9 | 13 |
| THC | 12 | 9 | 15 | 21 | 13 | 10 |
| CBD | 10 | 10 | 11 | 10 | 10 | 10 |
| CBD:THC (24:1) | 17 | 13 | 17 | 25 | 18 | 9 |

The withdrawal threshold gives an indication of the amount of pain that an animal is in. For example an animal that withdraws its paw after only a small amount of force being applied shows that the animal is in more pain than one that only withdraws its paw when a far greater force is applied.

As can be seen from Table 3 and Figure 3 the sham animals, (where no nerve injury had taken place), maintained a high withdrawal threshold of around 35-40 g over the entire testing period. When these data are compared to the other test animals, which all had CCI, it can be seen that the CCI caused the animals to feel more pain.

In the CCI animals treated with vehicle the withdrawal threshold was around 10-12 g over the entire testing period.

The three test articles pure THC, pure CBD and cannabinoid-containing plant extract containing CBD:THC (24:1) were shown to produce very different results.

As can be seen in Table 3 and Figure 3, the pure THC increased the withdrawal threshold over that of the vehicle treated animal 90 minutes after administration. The pain relieving effect of the pure THC reached a peak of 20 g after 120 minutes after which time the withdrawal threshold decreased back to that of the vehicle treated animals.

As was shown in the thermal hyperalgesia tests, the pure CBD did not produce any pain relieving effects over the study period, as the withdrawal threshold was very similar to that of the vehicle treated animal.

The withdrawal threshold of the animals treated with the cannabinoid-containing plant extract containing CBD:THC (24:1) showed an increase in withdrawal threshold 60 minutes after the test article was administered. The withdrawal threshold reached a peak after 120 minutes in these animals of 26 g, which then declined to a similar withdrawal latency as that shown by the vehicle treated animals after 180 minutes.

The data produced by both acute studies shows that the acute administration of a cannabinoid-containing plant extract containing CBD:THC at a ratio of 24:1 is effective at relieving pain caused by chronic constriction injury of the sciatic nerve in both models of neuropathic pain.

### 3. Effect of repeated administration of test article on thermal hyperalgesia

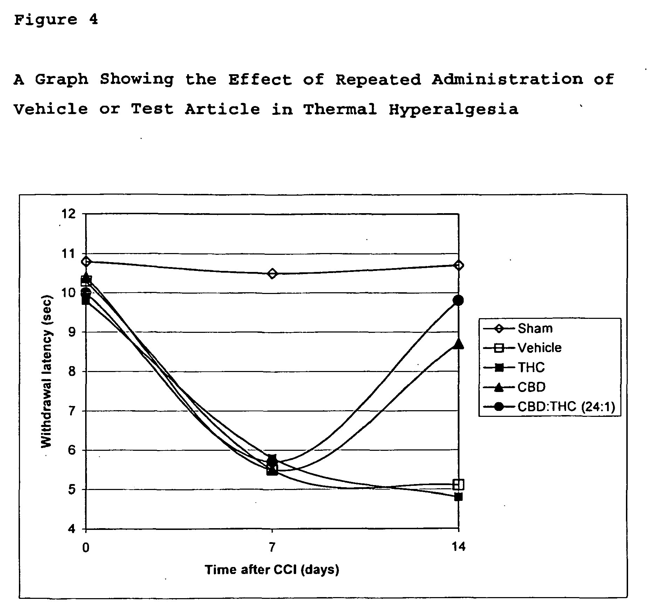

Table 4 below details the data produced by the repeated administration of the vehicle to the sham animals or administration of vehicle or test article to animals with CCI. Test article or vehicle was administered 7 days after the chronic constriction injury was applied and was administered once a day for a further seven days. Figure 4 describes these data graphically.

**Table 4:**

| **Test Article** | **Time after CCI (days)** | | |
|---|---|---|---|
| | 0 (prior to CCI) | 7 (before drug treatment) | 14 (after 7 days of drug treatment) |
| | **Withdrawal latency (seconds)** | | |
| Sham | 10.8 | 10.5 | 10.7 |
| Vehicle | 10.3 | 5.5 | 5.1 |
| THC | 9.8 | 5.8 | 4.8 |
| CBD | 10.4 | 5.5 | 8.7 |
| CBD:THC (24:1) | 10.0 | 5.7 | 9.8 |

The withdrawal latency gives an indication of the amount of pain that an animal is in. For example when an animal withdraws its paw soon after the heat source is applied it infers that the animal is in more pain than one that withdraws its paw a longer time after the heat source was applied.

As can be seen from Table 4 and Figure 4 the sham animals, (where no nerve injury had taken place), maintained a high withdrawal latency of around 11 seconds at day 7 before the start of the treatment phase and at day 14 at the end of the drug treatment phase.

When the data for the sham animals taken at day 7, prior to the beginning of the drug treatment phase, are compared to the other test animals, which all had CCI, it can be seen that the CCI caused the animals to feel more pain. The withdrawal latency before the CCI was between 10 and 11 seconds for all animals, whereas after the CCI the withdrawal latency for all CCI animals decreased dramatically to around 5.5 seconds.

In the CCI animals treated with vehicle the withdrawal latency remained at around 5 seconds after a further 7 days as would be expected.

The three test articles pure THC, pure CBD and cannabinoid-containing plant extract containing CBD:THC (24:1) were shown to produce very different results.

As can be seen in Table 4 and Figure 4, the pure THC did not result in any pain relieving effect. The withdrawal latency decreased after 7 days treatment with the pure THC and as such cannot be considered to have any pain relieving effect during repeated administration. These data differ from that produced in the acute administration studies for the animals treated with pure THC and demonstrate that the pain relieving effect of THC after acute administration is only temporary.

Converse to the data produced in the acute administration tests, the pure CBD was able to produce a pain relieving effect after 7 days repeated administration. The effect of the repeated administration of pure CBD over seven days resulted in an increase in the withdrawal latency from 5 seconds to 8 seconds inferring that the pure CBD produced an analgesic effect.

The withdrawal latency of the animals treated with the cannabinoid-containing plant extract containing CBD:THC (24:1) also showed an increase after seven days of repeated administration. The withdrawal latency increased from 5 seconds to 10 seconds after the repeated administration of the test article. The level of withdrawal latency reached a similar level to that experienced by the animals prior to the CCI.

### 4. Effect of repeated administration of test article on mechanical allodynia

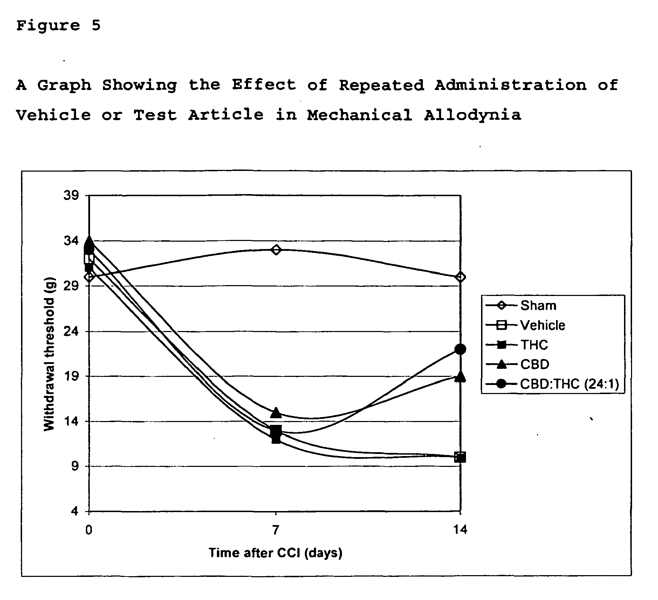

Table 5 below details the data produced by the repeated administration of the vehicle to the sham animals or administration of vehicle or test article to animals with CCI. Test article or vehicle was administered 7 days after the chronic constriction injury was applied and was administered once a day for a further seven days. Figure 5 describes these data in a graphical way.

**Table 5:**

| **Test Article** | **Time after CCI (days)** | | |
|---|---|---|---|
| | 0 (prior to CCI) | 7 (before drug treatment) | 14 (after 7 days of drug treatment) |
| | **Withdrawal threshold (g)** | | |
| Sham | 30 | 33 | 30 |
| Vehicle | 32 | 13 | 10 |
| THC | 31 | 12 | 10 |
| CBD | 34 | 15 | 19 |
| CBD:THC (24:1) | 33 | 13 | 22 |

The withdrawal threshold gives an indication of the amount of pain that an animal is in. For example an animal that withdraws its paw after only a small amount of force being applied shows that the animal is in more pain than one that only withdraws its paw when a far greater force is applied.

As can be seen from Table 5 and Figure 5 the sham animals, (where no nerve injury had taken place), maintained a high withdrawal threshold of around 30 g at day 7 before the start of the treatment phase and at day 14 at the end of the drug treatment phase.

When the data for the sham animals taken at day 7, prior to the beginning of the drug treatment phase, are compared to the other test animals, which all had CCI, it can be seen that the CCI caused the animals to feel more pain. The withdrawal threshold before the CCI was between 30 and 33 g for all animals, whereas after the CCI the withdrawal threshold for all CCI animals decreased dramatically to around 13 g.

In the CCI animals treated with vehicle the withdrawal latency decreased after the seven days of test article administration to 10 g inferring that as the time after the injury increased the amount of pain that the animals were experiencing also increased.

The three test articles pure THC, pure CBD and cannabinoid-containing plant extract containing CBD:THC (24:1) were again shown to produce very different results.

As can be seen in Table 5 and Figure 5, the pure THC did not result in any pain relieving effect. The withdrawal threshold decreased from 12 g to 10 g after 7 days treatment with the pure THC and as such cannot be considered to have any pain relieving effect during repeated administration. These data differ from that produced in the acute administration studies for the animals treated with pure THC and demonstrate that the pain relieving effect of THC after acute administration is only transitory.

Contrary to the data produced in the acute administration tests the pure CBD was able to produce a pain relieving effect after 7 days repeated administration. The effect of the repeated administration of pure CBD over seven days resulted in an increase in the withdrawal threshold from 15 g to 19 g inferring that the pure CBD produced an analgesic effect.

The withdrawal threshold of the animals treated with the cannabinoid-containing plant extract containing CBD:THC (24:1) also showed an increase after seven days of repeated administration. The withdrawal threshold increased from 13 g to 22 g after the repeated administration of the test article.

These data verify the conclusions made in the acute administration studies that the cannabinoid-containing plant extract containing CBD:THC at a ratio of 24:1 is more effective at relieving neuropathic pain than the purified cannabinoids THC or CBD alone.

To summarise the four different experiments Tables 6 and 7 below detail the effect of each test article in each test when compared to the vehicle.

Table 6 shows the mean pain relieving effect of the test article when compared to the vehicle as a percentage.

**Table 6:**

| | Thermal Hyperalgesia | Mechanical Allodynia |
|---|---|---|
| THC | 47% | 14% |
| CBD | -4% | -13% |
| CBD:THC (24:1) | 58% | 41% |

Table 7 shows the difference between the pain relieving values before and after treatment with the test article.

**Table 7:**

| | Thermal Hyperalgesia (sec) | Mechanical Allodynia (g) |
|---|---|---|
| Vehicle | -0.4 | -3 |
| THC | -1.0 | -2 |
| CBD | 3.2 | 4 |
| CBD:THC (24:1) | 4.1 | 9 |

### Conclusions:

In the acute administration tests the cannabinoid-containing plant extract that comprised the cannabinoids CBD and THC proved to be clearly more efficacious at the relief of neuropathic pain than either of the pure compounds THC and CBD. Pure THC was shown to have a pain relieving effect but this effect was short-lived and the amount of relief when compared to the vehicle treated animals was lower than that of the cannabinoid-containing plant extract.

In the repeated administration tests the cannabinoid-containing plant extract that comprised the cannabinoids CBD and THC again provided a more effective relief of neuropathic pain than either of the pure compounds THC and CBD. Interestingly rather than the pure THC that was shown to produce pain relief in the acute administration experiments, pure CBD produced a pain relieving effect after repeated administration of the drug. The summary tables above show that this effect was not as effective as the cannabinoid-containing plant extract that comprised the cannabinoids CBD and THC.

The reason why the cannabinoid-containing plant extract that comprised the cannabinoids CBD and THC are more efficacious than the pure compounds at the relief of neuropathic pain may be due to a synergistic effect of the two cannabinoids. However, it is possible that the other compounds that exist as part of the cannabinoid-containing plant extract, as described in Table 1, play a part in the mechanism of action of the relief of neuropathic pain by cannabinoids.

These data show that overall the cannabinoid-containing plant extract that comprised the cannabinoids CBD and THC are more suitable for use in the treatment of neuropathic pain.

### Example 2:

### Effect of concomitant administration of analgesic medication with a cannabinoid-containing plant extract in the treatment of neuropathic pain

A six week double blind, randomised, parallel group, placebo-controlled study of different cannabis based medicine extracts (CBME) was undertaken. The test articles that were studied were CBME THC:CBD (1:1) and matching placebo.

The study population were male or female patients aged 18 years or above, who have peripheral neuropathic pain characterised by allodynia. For inclusion in the study patients were required to have a history of at least 6 months duration of pain due to a clinically identifiable peripheral nerve lesion and were able to demonstrate mechanical allodynia as well as impairment of sensation within the territory of affected nerves and evidences of sensory derangement on clinical examination.

A baseline pain score of at least 4 on the Numerical rating Scale (NRS) for spontaneous pain on at least four of seven days in the baseline week was also required for eligibility of the study. Also required was a stable medication regimen of analgesics for at least two weeks prior to the study commencing.

The study medication was to be maintained concomitantly with the patient's existing medication throughout the study period. A summary of all medications taken by patients in the trial are listed below in Table 8:

**Table 8:**

| **Patient's Existing Medication** | **No. of patients in THC:CBD (1:1) group (%)** | **No. of patients in Placebo group (%)** |
|---|---|---|
| Natural opium alkaloids | 20 (31.7) | 32 (51.6) |
| Anti-epileptics | 20 (31.7) | 18 (29.0) |
| Non-selective monoamine reuptake inhibitors | 11 (17.5) | 19 (30.6) |
| Opioids | 11 (17.5) | 8 (12.9) |
| Anilides | 9 14.3) | 8 (12.9) |
| Diphenylpropylamine derivatives | 9 (14.3) | 6 (9.7) |
| Acetic acid derivatives and related substances | 4 (6.3) | 6 (9.7) |
| Platelet aggregation inhibitors excluding heparin | 8 (12.7) | 2 (3.2) |
| Carboxamide derivatives | 5 (7.9) | 3 (4.8) |
| Propionic acid derivatives | 3 (4.8) | 4 (6.5) |
| Salicylic acid derivatives | 2 (3.2) | 3 (4.8) |
| Local anaesthetics | 2 (3.2) | 2 (3.2) |
| Non-steroidal anti-inflammatory or anti-rheumatic compounds | 1 (1.6) | 2 (3.2) |
| Coxibs | 2 (3.2) | 1 (1.6) |
| Topical non-steroidal anti-inflammatory compounds | 1 (1.6) | 1 (1.6) |
| Opium alkaloids and derivatives | 1 (1.6) | 1 (1.6) |
| Anaesthetics for topical use | 1 (1.6) | 0 |
| Drugs used in opioid | 1 (1.6) | 0 |
| dependence | | |
| Hydantoin derivatives | 1 (1.6) | 0 |
| Oripavine derivatives | 1 (1.6) | 0 |
| Phenylpiperidine derivatives | 1 (1.6) | 0 |

### Results:

Table 9 shows a summary of the Neuropathic Pain Scale Total Scores in the Intention to Treat Population.

**Table 9:**

| | **THC:CBD (27mg/ml: 25mg/ml)** | **Placebo** |
|---|---|---|
| **Baseline (Visit 2)** | 61.1 | 62.4 |
| **Visit 4** | 50.9 | 60.4 |
| **Change from baseline** | -9.7 | -2.0 |

The data detailed above shows that there was a greater change from baseline in the group treated with the THC:CBD than with placebo. Statistical analysis was performed on the data and a p-value of 0.007 was obtained showing a statistically significant improvement of symptoms in the study medication treated group.

These data show that the concomitant administration of one or more analgesic drugs with a cannabinoid-containing plant extract comprising the cannabinoids CBD and THC are more efficacious in the relief of neuropathic pain than the treatment with the one or more analgesic drugs alone.

## Claims

1. A cannabis based medicinal extract (CBME) comprising CBD and THC for use in the treatment of peripheral neuropathic pain, wherein the ratio of CBD to THC, by weight, is between 18:1 and 30:1.

2. A CBME as claimed in claim 1, for use in the treatment of sleep disturbance caused by peripheral neuropathic pain.

3. A CBME as claimed in claim 1 or claim 2, with a longer profile of pain relief than pure THC for use as claimed in claim 1 or claim 2.

4. A CBME as claimed in any of the preceding claims for acute administration for use as claimed in any of the preceding claims.

5. A CBME as claimed in any of claims 1 to 3, which produces pain relief after daily repeated administration for use as claimed in any of the preceding claims

6. A CBME as claimed in claim 4 or claim 5, which provides pain relief both after acute administration and after daily repeated administration for use as claimed in any of the preceding claims.

7. A CBME as claimed in any of the preceding claims, wherein the ratio of CBD:THC by weight is between 22:1 and 26:1 for use as claimed in any of the preceding claims

8. A CBME as claimed in any of the preceding claims, wherein the ratio of CBD:THC by weight is substantially 24:1 for use as claimed in any of the preceding claims

9. A CBME as claimed in any of the preceding claims, which is packaged such that delivery is targeted to an area selected from the group: sublingual; buccal; parenteral; oral; rectal, nasal; and the pulmonary system for use as claimed in any of the preceding claims

10. A CBME as claimed in claim 9, which is in the form selected from the group: gel; gel spray; tablet; liquid; capsule and for vaporisation for use as claimed in any of the preceding claims.

11. A CBME as claimed in any of the preceding claims, which is formulated as a ratioed product from:
a) a cannabis based medicinal extract which comprises THC at more than 90% of the total cannabinoid content in the extract; and
b) a cannabis based medicinal extract which comprises CBD at more than 90% of the total cannabinoid content in the extract for use as claimed in any of the preceding claims.

12. A CBME as claimed in any of the preceding claims in combination with one or more other medicinal substances for use as claimed in any of the preceding claims.

13. A CBME as claimed in claim 12, wherein the one or more other medicinal substances are one or more analgesic drugs for use as claimed in any of the preceding claims.

14. A CBME as claimed in claim 12, wherein the one or more other medicinal substances are taken from the group: one or more opiate or opiate related drugs; one or more anticonvulsant drugs; and one or more antidepressant drugs for use as claimed in any of the preceding claims.

15. A CBME as claimed in any of claims 12 to 14, for administration separately, simultaneously or sequentially to the one or more other drugs for use as claimed in any of the preceding claims.

## Patentansprüche

1. Cannabisbasierter medizinischer Extrakt (CBME), der umfasst: CBD und THC zur Verwendung in der Behandlung von peripheren neuropatischen Schmerzen, wobei das Gewichtsverhältnis von CBD zu THC zwischen 18:1 und 30:1 liegt.

2. CBME gemäß Anspruch 1 zur Verwendung in der Behandlung von Schlafstörungen, die durch periphere neuropatische Schmerzen verursacht werden.

3. CBME gemäß Anspruch 1 oder Anspruch 2, mit einem längeren Profil von Schmerzlinderung als reines THC zur Verwendung gemäß Anspruch 1 oder Anspruch 2.

4. CBME gemäß einem der vorangehenden Ansprüche für eine akute Verabreichung zur Verwendung gemäß einem der vorangehenden Ansprüche.

5. CBME gemäß einem der Ansprüche 1 bis 3, der eine Schmerzlinderung nach einer täglich wiederholten Verabreichung erzeugt, zur Verwendung gemäß einem der vorangehenden Ansprüche.

6. CBME gemäß Anspruch 4 oder Anspruch 5, der eine Schmerzlinderung sowohl nach einer akuten als auch nach einer täglich wiederholten Verabreichung zur Verfügung stellt, zur Verwendung gemäß einem der vorangehenden Ansprüche.

7. CBME gemäß einem der vorangehenden Ansprüche, wobei das Gewichtsverhältnis von CBD:THC zwischen 22:1 und 26:1 liegt, zur Verwendung gemäß einem der vorangehenden Ansprüche.

8. CBME gemäß einem der vorangehenden Ansprüche, wobei das Gewichtsverhältnis von CBD:THC im Wesentlichen 24:1 ist, zur Verwendung gemäß einem der vorangehenden Ansprüche.

9. CBME gemäß einem der vorangehenden Ansprüche, der so verpackt ist, dass er gezielt einem der folgenden Gebiete zugeführt wird: dem sublingualen, bukkalen, parenteralen, oralen, rektalen, nasalen und pulmonalen System, zur Verwendung gemäß einem der vorangehenden Ansprüche.

10. CBME gemäß Anspruch 9, der eine aus der folgenden Gruppe ausgewählte Gestalt hat: Gel, Gel-Spray, Tablette, Flüssigkeit, Kapsel und zum Aufdampfen, zur Verwendung gemäß einem der vorangehenden Ansprüche.

11. CBME gemäß einem der vorangehenden Ansprüche, der als ein in ein Verhältnis gebrachtes Produkt aus den folgenden formuliert wird:
a) einem cannabisbasierten medizinischen Extrakt, der umfasst: THC bei mehr als 90 % des gesamten cannabinoiden Inhalts in dem Extrakt; und
b) einem cannabisbasierten medizinischen Extrakt, der umfasst: CBD bei mehr als 90 % des gesamten cannabinoiden Inhalts in dem Extrakt, zur Verwendung gemäß einem der vorangehenden Ansprüche.

12. CBME gemäß einem der vorangehenden Ansprüche in Kombination mit einer oder mehreren anderen medizinischen Substanzen, zur Verwendung gemäß einem der vorangehenden Ansprüche.

13. CBME gemäß Anspruch 12, wobei die eine oder mehreren anderen medizinischen Substanzen eine oder mehrere analgetische Drogen sind, zur Verwendung gemäß einem der vorangehenden Ansprüche.

14. CBME gemäß Anspruch 12, wobei die eine oder mehreren anderen medizinischen Substanzen aus der folgenden Gruppe genommen werden: ein oder mehrere Opiate oder opiatähnliche Drogen; eine oder mehrere antikonvulsive Drogen; und eine oder mehrere antidepressive Drogen, zur Verwendung gemäß einem der vorangehenden Ansprüche.

15. CBME gemäß einem der Ansprüche 12 bis 14 zur getrennten, gleichzeitigen oder zu der einen oder den mehreren Drogen nachfolgenden Verabreichung zur Verwendung gemäß einem der vorangehenden Ansprüche.

## Revendications

1. Extrait médicinal à base de cannabis (CBME) comprenant du CBD et du THC pour une utilisation dans le traitement de la douleur neuropathique périphérique, dans lequel le rapport du CBD sur le THC, en poids, se situe entre 18/1 et 30/1.

2. CMBE selon la revendication 1, pour une utilisation dans le traitement des troubles du sommeil provoqués par une douleur neuropathique périphérique.

3. CBME selon la revendication 1 ou la revendication 2, présentant un profil plus long de soulagement de la douleur que le THC pur, pour une utilisation selon la revendication 1 ou la revendication 2.

4. CBME selon l'une quelconque des revendications précédentes, pour une administration aiguë pour une utilisation selon l'une quelconque des revendications précédentes.

5. CBME selon l'une quelconque des revendications 1 à 3, qui produit un soulagement de la douleur après une administration quotidienne répétée pour une utilisation selon l'une quelconque des revendications précédentes.

6. CBME selon la revendication 4 ou la revendication 5, qui fournit un soulagement de la douleur à la fois après une administration aiguë et après une administration quotidienne répétée pour une utilisation selon l'une quelconque des revendications précédentes.

7. CBME selon l'une quelconque des revendications précédentes, dans lequel le rapport de CBD/THC en poids se situe entre 22/1 et 26/1 pour une utilisation selon l'une quelconque des revendications précédentes.

8. CBME selon l'une quelconque des revendications précédentes, dans lequel le rapport de CBD/THC en poids est pratiquement de 24/1 pour une utilisation selon l'une quelconque des revendications précédentes.

9. CBME selon l'une quelconque des revendications précédentes, qui est conditionné de telle manière que la délivrance est ciblée sur une zone choisie dans le groupe : sublinguale ; buccale ; parentérale ; orale ; rectale ; nasale ; et le système pulmonaire pour une utilisation selon l'une quelconque des revendications précédentes.

10. CBME selon la revendication 9, qui est sous la forme choisie dans le groupe : gel ; spray de gel ; comprimé ; liquide ; capsule et pour une vaporisation pour une utilisation selon l'une quelconque des revendications précédentes.

11. CBME selon l'une quelconque des revendications précédentes, qui est formulé sous la forme d'un produit à rapports déterminés à partir :
a) d'un extrait médicinal à base de cannabis qui comprend du THC à plus de 90 % de la teneur totale en cannabinoïdes dans l'extrait ; et
b) d'un extrait médicinal à base de cannabis qui comprend du CBD à plus de 90 % de la teneur totale en cannabinoïdes dans l'extrait pour une utilisation selon l'une quelconque des revendications précédentes.

12. CBME selon l'une quelconque des revendications précédentes en combinaison avec une ou plusieurs autres substances médicinales pour une utilisation selon l'une quelconque des revendications précédentes.

13. CBME selon la revendication 12, où les une ou plusieurs autres substances médicinales sont un ou plusieurs médicaments analgésiques pour une utilisation selon l'une quelconque des revendications précédentes.

14. CBME selon la revendication 12, où les une ou plusieurs autres substances médicinales sont choisies dans le groupe : un ou plusieurs médicaments opiacés ou apparentés aux opiacés ; un ou plusieurs médicaments anticonvulsivants , et un ou plusieurs médicaments antidépresseurs pour une utilisation selon l'une quelconque des revendications précédentes.

15. CBME selon l'une quelconque des revendications 12 à 14, pour une administration séparément, simultanément ou séquentiellement par rapport aux un ou plusieurs autres médicaments pour une utilisation selon l'une quelconque des revendications précédentes.
